**Europäisches Patentamt**

**European Patent Office**

**Office européen des brevets**

(19)

(11) Veröffentlichungsnummer: **0 009 743**
**B1**

(12) **EUROPÄISCHE PATENTSCHRIFT**

(45) Veröffentlichungstag der Patentschrift:
27.01.82

(51) Int. Cl.³: **C 07 C 143/66**, C 07 C 139/00

(21) Anmeldenummer: **79103590.0**

(22) Anmeldetag: **24.09.79**

(54) Verfahren zur Herstellung von 1-Amino-8-naphthol-4,6-disulfonsäure (K-Säure).

(30) Priorität: **06.10.78 DE 2843680**

(43) Veröffentlichungstag der Anmeldung:
**16.04.80 Patentblatt 80/8**

(45) Bekanntmachung des Hinweises auf die Patenterteilung:
**27.01.82 Patentblatt 82/4**

(84) Benannte Vertragsstaaten:
**BE CH DE FR GB IT NL**

(56) Entgegenhaltungen:
**EP-A-0 000 493**
**BE-A-865 480**
**DE-A-2 716 030**
**DE-A-2 727 345**

(73) Patentinhaber: **BAYER AG, Zentralbereich Patente,
Marken und Lizenzen, D-5090 Leverkusen 1, Bayerwerk
(DE)**

(72) Erfinder: **Behre, Horst, Dr., Im Hellsiefen 4,
D-5068 Odenthal (DE)**
Erfinder: **Blank, Heinz Ulrich, Dr., Am Geusfeld 35,
D-5068 Odenthal (DE)**
Erfinder: **Schössler, Willi, Dr., Hahnenweg 2,
D-5000 Köln 80 (DE)**

### Verfahren zur Herstellung von 1-Amino-8-naphthol-4,6-disulfonsäure (K-Säure)

Die vorliegende Erfindung betrifft ein Verfahren zur Herstellung von 1-Amino-8-naphthol-4,6-disulfonsäure (K-Säure) als Monoalkalisalz aus Naphthylamin-trisulfonsäure-Isomerengemischen durch alkalische Druckhydrolyse.

1-Amino-8-naphthol-4,6-disulfonsäure, die häufig auch als K-Säure bezeichnet wird, ist ein wichtiges Zwischenprodukt zur Herstellung von Farbstoffen (s. Ullmanns Enzyklopädie der technischen Chemie, 3. Aufl., 12. Band, S. 622).

Aus der BE-A-865 840 ist die alkalische Druckhydrolyse von Melansäure zu K-Säure und aus der DE-A-2 727 345 die alkalische Druckhydrolyse von T-Säure zu H-Säure bekannt. Ferner ist aus FIAT Final Report Nr. 1016, S. 42—44 und BIOS Final Report Nr. 1152 (Item Nr. 22, S. 105), bekannt, daß man K-Säure wie folgt herstellen kann:

Naphthalin wird bei 30 bis 35°C unter gleichzeitigem Einlaufen von 65%igem Oleum in Schwefelsäuremonohydrat (100%ige $H_2SO_4$) eingetragen und die Reaktionsmischung eine Stunde bei 50°C, eine Stunde bei 70°C und sieben Stunden bei 90°C gehalten. Das erhaltene Naphthalintrisulfonsäure-Isomerengemisch wird mit Mischsäure nitriert. Nach Verdünnung mit Wasser, Austreiben der nitrosen Gase und Abtrennung der Schwefelsäure als Calciumsulfat wird das Isomerengemisch der Nitronaphthalintrisulfonsäuren mit Eisen reduziert und dann gelöste Eisensalze mit Magnesiumoxid gefällt und abgetrennt. Durch Zugabe von Salzsäure wird zunächst das saure Calciumsalz der T-Säure (1-Naphthylamin-3,6,8-trisulfonsäure) ausgefällt, das abfiltriert und mit Salzwasser gewaschen wird. Aus dem Filtrat wird dann die rohe 1-Naphthylamin-4,6,8-trisulfonsäure (Naphthamintrisäure K, Melansäure) durch Zugabe von Steinsalz und Salzsäure als saures Dinatriumsalz abgeschieden, das mit Salzwasser gewaschen wird. Zur Reinigung wird das Rohprodukt in Wasser angeschlämmt, mit Soda in Lösung gebracht, die Lösung zur Entfernung von Calciumcarbonat filtriert, aufkonzentriert und die reine Melansäure als saures Dinatriumsalz durch Zugabe von Salzsäure ausgefällt und abfiltriert.

Das saure Melansäure-dinatriumsalz wird mit Ätznatron und Wasser unter Druck bei 170°C im Verlaufe von 12 Stunden umgesetzt. Anschließend wird durch Zugabe von Salzsäure und Wasser die K-Säure als saures Mononatriumsalz durch Filtration gewonnen.

Bei diesem Verfahren ist nachteilig, daß auf einer Zwischenstufe zunächst die T-Säure als saures Calciumsalz und anschließend die Melansäure als saures Dinatriumsalz abgetrennt und letzteres durch Umlösung gereinigt werden muß. Dabei fällt ein Abwasser an, dessen Aufarbeitung äußerst schwierig und kostspielig ist, da es neben organischen Bestandteilen auch große Mengen Natriumchlorid, Calciumchlorid und Salzsäure enthält.

Das abgeschiedene saure T-Säure-Calciumsalz muß noch durch Umsetzung mit Natriumcarbonat in das Trinatriumsalz überführt werden, wenn es beispielsweise nach FIAT Final Report Nr. 1016, S. 33—36, in die 1-Amino-8-naphthol-3,6-disulfonsäure (H-Säure) überführt werden soll

Die Abscheidung der Melansäure als saures Dinatriumsalz ist unvollständig und die Umlösung des Rohproduktes führt zu weiteren Ausbeuteverlusten. Das abgeschiedene feuchte Melansäuresalz enthält noch Natriumchlorid und anhaftende Salzsäure. Diese können nur unter Verlust von Melansäure ausgewaschen werden. Wenn man beide Komponenten in der Melansäure beläßt, erfordert die anschließende Umsetzung mit Ätznatron und Wasser einen erhöhten Verbrauch an Ätznatron, um die nötige Laugenkonzentration im Reaktionsgemisch einzustellen.

Bei der Ansäuerung der alkalischen Reaktionslösung mit Salzsäure werden zusammen mit der K-Säure erhebliche Mengen an 1-Amino-6-naphthol-4,8-disulfonsäure (Iso-K-Säure) abgeschieden, wie eine Nacharbeitung ergeben hat (vgl. Beispiel 5).

Dieses Nebenprodukt kann nur durch aufwendige Reinigungsoperationen entfernt werden. Außerdem fällt auch an dieser Stelle ein Abwasser an, dessen Aufarbeitung äußerst schwierig und kostspielig ist, da es neben den organischen Bestandteilen auch große Mengen Natriumchlorid und Salzsäure enthält.

Es wurde nun ein Verfahren zur Herstellung von 1-Amino-8-naphthol-4,6-disulfonsäure-monoalkalisalzen (K-Säure-monoalkalisalzen) durch alkalische Druckhydrolyse von 1-Naphthylamin-4,6,8-trisulfonsäure (Melansäure) gefunden, das dadurch gekennzeichnet ist, daß man ein Naphthylamintrisulfonsäure-Isomeren-Gemisch, wie es bei der technischen Herstellung von Melansäure anfällt und das über 40 Gew.-% 1-Naphthylamin-4,6,8-trisulfonsäure (bezogen auf die Gesamtmenge diazotierbarer Substanzen, gerechnet mit Molekulargewicht 383) enthält, und/oder deren Salze mit Alkalihydroxid-Lösung bei erhöhtem Druck und erhöhter Temperatur umsetzt und anschließend durch Ansäuern und Kristallisation die 1-Amino-8-naphthol-3,6-disulfonsäure (H-Säure) und die 1-Amino-8-naphthol-4,6-disulfonsäure jeweils in Form ihrer Monoalkalisalze isoliert.

Bevorzugt werden solche Gemische eingesetzt, die 45 bis 65 Gew.-% 1-Naphthylamin-4,6,8-trisulfonsäure enthalten. Ein besonders bevorzugt einzusetzendes Naphthylamintrisulfonsäure-Gemisch enthält

| | |
|---|---|
| 50 bis 60 Gew.-% | 1-Naphthylamin-4,6,8-trisulfonsäure |
| 25 bis 35 Gew.-% | 1-Naphthylamin-3,6,8-trisulfonsäure |

| | |
|---|---|
| 5 bis 10 Gew.-% | 2-Naphthylamin-4,6,8-trisulfonsäure |
| 0,5 bis 2 Gew.-% | 1-Naphthylamin-2,5,7-trisulfonsäure |
| 0,1 bis 2 Gew.-% | 1-Naphthylamin-3,5,7-trisulfonsäure |
| 0,1 bis 0,5 Gew.-% | 2-Naphthylamin-3,6,8-trisulfonsäure |

Solche Gemische können beispielsweise erhalten werden, indem man Naphthalin trisulfiert, das entstandene Gemisch nitriert und dann das vorliegende Nitronaphthalintrisulfonsäure-Gemisch reduziert. Diese Reaktionen können gemäß der eingangs beschriebenen Arbeitsweise nach FIAT Final Report Nr. 1016, S. 42 bis 44, oder auf beliebige andere Weise durchgeführt werden.

Das Naphthylamintrisulfonsäure-Gemisch kann die Säuren in freier Form, in Form von neutralen Salzen oder in Form von sauren Salzen enthalten. Auch Gemische, die freie Säuren und Salze enthalten, können verwendet werden. Sofern die Naphthylamintrisulfonsäuren ganz oder teilweise als Salze vorliegen, sind die Alkali- und Erdalkalisalze, insbesondere die Natrium- und Kaliumsalze, bevorzugt. Ganz besonders bevorzugt sind Naphthylamintrisulfonsäure-Gemische, welche die Säuren in Form von Trinatriumsalzen enthalten.

Das Naphthylamintrisulfonsäure-Gemisch kann neben den Naphthylamintrisulfonsäuren bzw. deren Salzen noch weitere Produkte enthalten. Solche Produkte können insbesondere Nebenprodukte, Zersetzungsprodukte oder nicht umgesetzte Zwischenprodukte aus den Herstellungsstufen für Naphthylamintrisulfonsäuren sein, beispielsweise Naphthalin-di-, -tri- und -tetra-sulfonsäuren, Nitronaphthalin-mono-, -di- und -trisulfonsäuren, Naphthylamin-mono- und -disulfonsäuren, z. B. 1-Naphthylamin-4,6- und -4,8-disulfonsäure, sowie 2-Naphthylamin-4,8-disulfonsäure, weiterhin Dinaphthylsulfon-sulfosäuren und deren Amino- und Nitroderivate, sowie Oxidationsprodukte des Naphthalins und/oder der Naphthalinsulfonsäuren, die bei der Sulfierung und/oder der Nitrierung gebildet werden können.

Das Naphthylamintrisulfonsäure-Gemisch kann in fester Form, als wäßrige Lösung oder als Suspension mit einem Gehalt von beispielsweise 20 bis 50 Gew.-%, vorzugsweise 25 bis 40 Gew.-%, gerechnet als freie Säure mit dem Molekulargewicht 383, eingesetzt werden.

Als Alkalihydroxidlösungen für das erfindungsgemäße Verfahren kommen insbesondere wäßrige Kali- oder Natronlauge in Frage. Bevorzugt kommt Natronlauge zum Einsatz. Pro Mol diazotierbare Substanz (gerechnet mit Molekulargewicht 383 = Melansäure) können beispielsweise 3,5 bis 12 Mol Alkalihydroxid eingesetzt werden. Besonders bevorzugt ist der Einsatz von 5 bis 8 Mol Alkalihydroxid pro Mol diazotierbare Substanz. Die Konzentration von Alkalihydroxid im Reaktionsgemisch kann beispielsweise 10 bis 50 Gew.-% (bezogen auf die Summe Alkalihydroxid + gesamtes Wasser) betragen. Bevorzugt ist diese Konzentration 20 bis 40gew.-%ig.

Die Umsetzung kann bei Temperaturen von 150 bis 250°C, vorzugsweise bei 160 bis 220°C in einem geschlossenen Gefäß durchgeführt werden. Der sich dabei einstellende Druck ist im allgemeinen völlig ausreichend, um das erfindungsgemäße Verfahren in befriedigender Weise durchzuführen. Selbstverständlich kann man das erfindungsgemäße Verfahren auch bei anderen Drucken durchführen als denjenigen, die sich im geschlossenen Gefäß von selbst einstellen. Beispielsweise sind für das erfindungsgemäße Verfahren Drucke im Bereich von 5 bis 50 bar möglich.

Die Reaktionszeit hängt im wesentlichen von der Reaktionstemperatur und der Alkalihydroxid-Konzentration ab. Sie ist bei relativ hohen Reaktionstemperaturen und bei relativ hohen Alkalihydroxid-Konzentrationen kürzer und bei relativ niedrigen Reaktionstemperaturen und relativ niedrigen Alkalihydroxid-Konzentrationen länger und beträgt im allgemeinen 10 Minuten bis 25 Stunden. Beispielsweise werden bei einer Reaktionstemperatur von ca. 200°C und einer Alkalihydroxid-Konzentration von 30 Gew.-% mit einer Reaktionszeit von 90 Min. gute Ergebnisse erhalten, während man bei einer Reaktionstemperatur von ca. 170°C ca. 17 Stunden benötigt.

Bei der Durchführung des erfindungsgemäßen Verfahrens ist es wesentlich, daß während der gesamten Reaktion günstige Alkalihydroxid-Konzentrationen vorliegen. Man arbeitet deshalb vorzugsweise so, daß man das Naphthylamintrisulfonsäure-Isomerengemisch in Form einer schwach alkalischen, wäßrigen Lösung und die Hauptmenge der Alkalihydroxidlösung simultan im Verlauf von beispielsweise 5 bis 30 Minuten, vorzugsweise 10 bis 20 Minuten, in eine Vorlage aus wenig Alkalihydroxidlösung einpumpt und das Gemisch anschließend ausreagieren läßt. Die Ausgangsstoffe werden am zweckmäßigsten mit einer solchen Temperatur in das Reaktionsgefäß eingebracht, daß man nach Freiwerden der Mischungs- und gegebenenfalls der Neutralisationswärme die gewünschte Reaktionstemperatur vorliegen hat. Man kann die Ausgangsstoffe auch bei tieferen Temperaturen zusammenbringen und im Reaktionsgefäß auf die gewünschte Reaktionstemperatur erhitzen.

Nach Beendigung der Reaktion und vor dem Abscheiden der 1-Amino-8-naphthol-3,6-disulfonsäure (H-Säure) und der 1-Amino-8-naphthol-4,6-disulfonsäure (K-Säure) ist es vorteilhaft, das Reaktionsgemisch zu kühlen und/oder mit Wasser zu verdünnen. Man kan beispielsweise auf Temperaturen im Bereich 20 bis 150°C, vorzugsweise auf Temperaturen im Bereich 80 bis 120°C, abkühlen. Die gegebenenfalls zuzusetzende Wassermenge richtet sich nach den Reaktionsbedingungen, z. B. der Art des Alkalihydroxids, dessen Menge und Konzentration. Es ist vorteilhaft, die Wassermenge so zu wählen, daß das bei der Reaktion gebildete Alkalisulfit gelöst wird.

Die Abscheidung der H-Säure und K-Säure als Monoalkalisalze kann durch Ansäuern der

3

Reaktionsmischung mit Mineralsäuren erfolgen. Vorzugsweise verwendet man hierzu Schwefelsäure. Man gibt so viel Mineralsäure zu, daß sich die schwerlöslichen Monoalkalisalze der H-Säure und K-Säure bilden. Durch entsprechende Wahl der Konzentration der Mineraısäure und/oder durch Zugabe von Wasser vor und/oder während der Zugabe der Mineralsäure wird zweckmäßigerweise dafür gesorgt, daß das sich bildende anorganische Salz, z. B. das Natriumsulfat oder Kaliumsulfat, nicht ausfällt. Man kann beispielsweise gute Ergebnisse erhalten, wenn man zur Abscheidung der H-Säure und der K-Säure als Monoalkalisalze einen pH-Wert im Bereich 0 bis 4, vorzugsweise 0,5 bis 2,5, einstellt und durch Verdünnung mit Wasser und/oder durch entsprechende Wahl der Konzentration der Mineralsäure, bezogen auf das Gewicht des in der Druckhydrolyse vorliegenden Gemisches, die 0,1- bis 5fache, vorzugsweise die 0,5- bis 2fache Menge Wasser einbringt.

Die Isolierung der Monoalkalisalze der H-Säure und K-Säure kann auf verschiedene Weise erfolgen. Zwei bevorzugte Varianten sind im folgenden detailliert angegeben:

## Variante 1

Die angesäuerte Reaktionsmischung wird bei Temperaturen im Bereich von 40 bis 100°C, bevorzugt bei 50 bis 90°C, insbesondere bei 60 bis 80°C, einige Zeit, beispielsweise 1 bis 4 Stunden, gerührt. Dabei scheidet sich das Monoalkalisalz der H-Säure ab und kann auf übliche Weise, beispielsweise durch Filtration, abgetrennt werden. Das abgetrennte Produkt wird mit wäßriger Alkalisulfatlösung und/oder mit Wasser gewaschen und getrocknet, beispielsweise im Vakuum. Die Waschlaugen werden in den folgenden Ansatz anstelle von Wasser recyclisiert. Das Filtrat der H-Säure-Abscheidung wird bei Temperaturen im Bereich von −5 bis 40°C, bevorzugt bei 5 bis 25°C, einige Zeit, beispielsweise 2 bis 24 Stunden, gerührt. Dabei scheidet sich das Monoalkalisalz der K-Säure ab und kann auf übliche Weise, beispielsweise durch Filtration, abgetrennt werden. Das abgetrennte Produkt wird mit wäßriger Alkalisulfat-Lösung und/oder mit Wasser gewaschen und getrocknet, beispielsweise im Vakuum. Die Waschlaugen werden in den folgenden Ansatz anstelle von Wasser recyclisiert.

## Variante 2

Die angesäuerte Reaktionsmischung wird bei Temperaturen im Bereich von −5 bis 40°C, bevorzugt bei 5 bis 25°C, einige Zeit gerührt, beispielsweise 2 bis 24 Stunden. Dabei scheidet sich ein Gemisch der Monoalkalisalze der H-Säure und K-Säure ab und kann auf übliche Weise, beispielsweise durch Filtration, abgetrennt werden. Das abgetrennte Produkt wird mit wäßriger Alkalisulfatlösung gewaschen und die Waschlauge in den folgenden Ansatz anstelle von Wasser recyclisiert. Das abgetrennte H-Säure/K-Säure-Gemisch wird in Wasser und/oder wäßriger Alkalisulfat-Lösung gegebenenfalls unter Zusatz geringer Mengen Mineralsäuren, vorzugsweise Schwefelsäure, suspendiert, das Monoalkalisalz der K-Säure bei Temperaturen im Bereich von 40 bis 100°C, vorzugsweise 50 bis 90°C, insbesondere 60 bis 80°C im Verlaufe einiger Zeit, beispielsweise 1 bis 10 Stunden in Lösung gebracht und das Monoalkalisalz der H-Säure durch Filtration, Wäsche mit wäßriger Alkalisulfat-Lösung und gegebenenfalls Wasser und Trocknung, beispielsweise im Vakuum, erhalten. Die Waschlauge wird in den folgenden Ansatz anstelle von Wasser bei der Anschlämmung des H-Säure/K-Säure-Gemisches recyclisiert. Das nach der H-Säure-Abtrennung verbleibende Filtrat wird bei Temperaturen im Bereich von −5 bis 40°C, bevorzugt 5 bis 25°C, einige Zeit, beispielsweise 2 bis 24 Stunden gerührt und das Monoalkalisalz der K-Säure durch Filtration, Wäsche mit wäßriger Alkalisulfat-Lösung und/oder Wasser und Trocknung, beispielsweise im Vakuum, erhalten. Die Waschlauge wird in den folgenden Ansatz anstelle von Wasser bei der Anschlämmung des H-Säure/K-Säure-Gemisches recyclisiert.

Zur vollständigen Entfernung von Schwefeldioxid ist es vorteilhaft, nach Einstellung der Fällungsbedingungen und vor der Abtrennung der Monoalkalisalze der H-Säure und K-Säure das angesäuerte und verdünnte Gemisch für einige Zeit, beispielsweise 0,5 bis 2 Stunden, bei 80 bis 100°C zu halten oder das Schwefeldioxid mit einem inerten Gas, z. B. Stickstoff, auszublasen.

Nach dem erfindungsgemäßen Verfahren kann man die gebildeten Monoalkalisalze der H-Säure und K-Säure in hohen Ausbeuten und hohen Reinheiten (z. B. 97−99%, bezogen auf insgesamt vorliegende organische Verbindungen) isolieren.

Dies ist überraschend, da sich in dem nach der Druckhydrolyse vorliegenden Gemisch ein große Zahl verschiedener Komponenten nachweisen lassen und nur ca. 30 bis 40% des eingesetzten Naphthalins in Form von H-Säure und K-Säure vorliegen. Insbesondere die schwerlösliche Iso-K-Säure (1-Amino-6-Naphthol-4,8-disulfonsäure) wird beispielsweise in dem isolierten Monoalkalisalz der K-Säure in wesentlich geringerer Menge gefunden, als beim Einsatz von reiner Melansäure bzw. reinem Melansäure-di-natriumsalz (s. Beispiel 5).

Das erfindungsgemäße Verfahren bietet gegenüber der bisher üblichen alkalischen Druckhydrolyse von abgeschiedener Melansäure erhebliche Vorteile und ist daher besonders wirtschaftlich. So entfallen beim erfindungsgemäßen Verfahren die Verfahrensstufen für die Abscheidung, Isolierung und Umlösung der T-Säure als saures Calciumsalz und der Melansäure als saures Dinatriumsalz. Für die alkalische Druckhydrolyse wird die gesamte T-Säure und Melansäure ausgenützt, so daß eine verbesserte Ausbeute an H-Säure und K-Säure, bezogen auf ursprünglich eingesetztes Naphthalin, resultiert.

Beim Einsatz der salzfreien Melansäure-Isomerengemisch-Lösung läßt sich im Vergleich zum Natriumchlorid- und Salzsäure-haltigen Melansäure-dinatriumsalz die alkalische Druckhydrolyse bei höheren Konzentrationen durchführen. Daraus resultiert bei gleicher Alkalikonzentration im Reaktionsgemisch eine Einsparung an Alkalihydroxid. Schließlich fällt beim erfindungsgemäßen Verfahren nur einmal Abwasser an, nämlich bei der Abscheidung der H-Säure und K-Säure. Dieses Abwasser enthält neben organischen Verbindungen nur das bei der Neutralisation entstehende anorganische Salz, z. B. Natriumsulfat, und wenig Mineralsäure, z. B. Schwefelsäure. Insgesamt sind die Abwasser- und Salzmengen gegenüber den bisherigen Verfahren sehr stark reduziert.

## Beispiel 1

In einem 2,7-l-Nickel-Autoklav werden 689 g eines Naphthylamintrisulfonsäure-Gemisches in Form der Trinatriumsalze (Gehalt 10,02 g Gesamtnitrit/100 g, 28,6 Gew.-% Melansäure MG 383; insgesamt 69 g Nitrit, 0,51 Mol Melansäure) folgender Zusammensetzung:

| | |
|---|---|
| 1-Naphthylamin-4,6,8-trisulfonsäure | 51,4% |
| 1-Naphthylamin-3,6,8-trisulfonsäure | 31,8% |
| 2-Naphthylamin-4,6,8-trisulfonsäure | 9,0% |
| 1-Naphthylamin-2,5,7-trisulfonsäure | 1,2% |
| 1-Naphthylamin-3,5,7-trisulfonsäure | 2,5% |
| 2-Naphthylamin-3,6,8-trisulfonsäure | 0,2% |

(%-Gehalte jeweils bezogen auf diazotierbare Substanz) das zusätzlich

| | |
|---|---|
| 0,6 Gew.-% | 2-Naphthylamin-4,8-disulfonsäure-dinatriumsalz, |
| 0,1 Gew.-% | 1-Naphthylamin-4,8-disulfonsäure-dinatriumsalz |
| 3,1 Gew.-% | Wasser |
| 1,4 Gew.-% | Natriumcarbonat |

und quantitativ nicht bestimmbare Mengen an Amino- und Nitroderivaten der Dinaphthylsulfon-sulfosäuren und an Oxidationsprodukten des Naphthalins und der Naphthalintrisulfonsäuren enthält und 435 g Wasser vorgelegt und auf 190°C aufgeheizt. In einem 1,3-l-Stahl-Autoklav werden 343 g 70gew.-%ige Natronlauge (6,0 Mol NaOH) auf 180°C aufgeheizt und mit Stickstoff in den 2,7-l-Autoklav gedrückt, wodurch bezogen auf das gesamte Wasser eine 30gew.-%ige Natronlauge entsteht. Dabei stellt sich eine Temperatur von 200°C ein. Die Reaktionsmischung wird 90 Minuten bei 200°C gehalten, so schnell wie möglich auf 100°C abgekühlt und mit 1800 g Wasser verdünnt. Die heiße Reaktionslösung wird pH-kontrolliert (pH 1 bis 1,5), mit ca. 1100 g 50gew.-%iger Schwefelsäure angesäuert, zur vollständigen Entfernung von Schwefeldioxid 1 Stunde unter Einleiten von Stickstoff bei 80°C gehalten, unter Verdampfungskühlung auf Raumtemperatur abgekühlt und 12 Stunden bei Raumtemperatur (20°C) gehalten. Das Produkt wird bei Raumtemperatur filtriert, mit insgesamt 600 g einer 15gew.-%igen wäßrigen Natriumsulfat-Lösung gewaschen, und im Vakuum bei 60°C getrocknet.

Die durch Diazotieren bestimmte Ausbeute beträgt 55%, bezogen auf den Nitrit-Gehalt des eingesetzten Naphthylamin-trisulfonsäure-Gemisches. Die Zusammensetzung des H-Säure/K-Säure-Mononatriumsalz-Gemisches wurde durch Hochdruck-Flüssigkeits-Chromatographie wie folgt ermittelt:

| | |
|---|---|
| K-Säure-Mononatriumsalz | 32,1 Gew.-% |
| H-Säure-Mononatriumsalz | 18,7 Gew.-% |
| Iso-K-Säure-Mononatriumsalz | 0,1 Gew.-% |
| W-Säure-Mononatriumsalz | 0 Gew.-% |
| Melansäure-Dinatriumsalz | 0,1 Gew.-% |
| T-Säure-Dinatriumsalz | 0,1 Gew.-% |
| Dioxi-K-Säure-Dinatriumsalz | 0 Gew.-% |
| Chromotrop-Säure-Dinatriumsalz | 0,4 Gew.-% |
| Wasser | 9,2 Gew.-% |
| Natriumsulfat | 39,0 Gew.-% |

Reaktionsprodukte aus den weiteren Naphthylamin-di- und -trisulfonsäuren und den weiteren Nebenprodukten sind in dem isolierten Produkt nicht enthalten.

5

150 g des K-Säure/H-Säure-Mononatriumsalz-Gemisches wurden in 350 ml Wasser angeschlämmt. Der pH-Wert wurde durch Zugabe von Schwefelsäure auf 1 bis 1,5 eingestellt und die Reaktionsmischung 2 Stunden bei 70°C gerührt. Das H-Säure-Mononatriumsalz wurde bei 70°C abfiltriert, mit insgesamt 50 ml einer 15%igen wäßrigen Natriumsulfatlösung gewaschen und im Vakuum bei 60°C getrocknet. Die Ausbeute beträgt bei Recyclisierung des Waschwassers 62%, bezogen auf T-Säure. Die H-Säure-Qualität wird durch Hochdruck-Flüssigkeits-Chromatographie wie folgt ermittelt:

| | |
|---|---|
| H-Säure-Mononatriumsalz | 34,7 Gew.-% |
| K-Säure-Mononatriumsalz | 1,2 Gew.-% |
| W-Säure-Mononatriumsalz | 0 Gew.-% |
| Iso-K-Säure-Mononatriumsalz | 0,1 Gew.-% |
| Chromotrop-Säure-Dinatriumsalz | 0,2 Gew.-% |
| Wasser | 12,4 Gew.-% |
| Natriumsulfat | 51,2 Gew.-% |

Durch erneutes Anschlämmen in kaltem Wasser (ca. 20°C) wurde das H-Säure-Mononatriumsalz in praktisch reiner Form erhalten.

| | |
|---|---|
| H-Säure-Mononatriumsalz | 88,1 Gew.-% |
| K-Säure-Mononatriumsalz | 0,2 Gew.-% |
| Wasser | 9,4 Gew.-% |
| Natriumsulfat | 2,0 Gew.-% |

Das Filtrat aus der H-Säure-Mononatriumsalz-Abtrennung wurde auf Raumtemperatur (ca. 20°C) kalt gerührt und 12 Stunden bei Raumtemperatur gehalten. Das K-Säure-Mononatriumsalz wurde abfiltriert, mit insgesamt 50 ml Eiswasser gewaschen und im Vakuum bei 60°C getrocknet.
Die Ausbeute beträgt bei Recyclisierung des Filtrates 70% bezogen auf Melansäure. Die K-Säure-Qualität wurde durch Hochdruck-Flüssigkeits-Chromatographie wie folgt ermittelt:

| | |
|---|---|
| K-Säure-Mononatriumsalz | 80,2 Gew.-% |
| H-Säure-Mononatriumsalz | 2,5 Gew.-% |
| Iso-K-Säure-Mononatriumsalz | 0 Gew.-% |
| W-Säure-Mononatriumsalz | 0 Gew.-% |
| Dioxi-K-Säure-Mononatriumsalz | 0 Gew.-% |
| Chromotrop-Säure-Dinatriumsalz | 0,1 Gew.-% |
| Melan-Säure-Dinatriumsalz | 0,1 Gew.-% |
| T-Säure-Dinatriumsalz | 0,1 Gew.-% |
| Wasser | 12,4 Gew.-% |
| Natriumsulfat | 4,3 Gew.-% |

Durch erneutes Umlösen wird das K-Säure-Mononatriumsalz in praktisch reiner Form erhalten.

## Beispiel 2

Es wurde wie in Beispiel 1 verfahren, jedoch wurde die Reaktionsmischung nach Entfernung des Schwefeldioxids 4 Stunden bei 70°C gehalten. Das H-Säure-Mononatriumsalz wurde bei 70°C filtriert, mit insgesamt 100 ml einer 15gew.-%igen wäßrigen Natriumsulfatlösung gewaschen und im Vakuum bei 60°C getrocknet. Die Ausbeute beträgt bei Recyclisierung des Waschwassers anstelle von Wasser in den nächsten Ansatz 61% bezogen auf T-Säure. Die H-Säure-Qualität entspricht derjenigen des Beispiels 1.
Das Filtrat wurde auf Raumtemperatur (ca. 20°C) kalt gerührt und 12 Stunden bei Raumtemperatur gehalten. Das K-Säure-Mononatriumsalz wurde abfiltriert, mit insgesamt 100 ml Eiswasser gewaschen und im Vakuum bei 60°C getrocknet. Die Ausbeute beträgt bei Recyclisierung des Waschwassers anstelle von Wasser in den nächsten Ansatz 70% bezogen auf Melansäure. Die K-Säure-Qualität entspricht derjenigen des Beispiels 1.

### Beispiele 3a bis 3d

Es wurde wie im Beispiel 1 verfahren, jedoch wurden folgende Reaktionsparameter variiert:

1. Molverhältnis Natronlauge zu Melansäure/T-Säure-Isomerengemisch in Form der Trinatriumsalze
2. Natronlauge-Konzentration bezogen auf gesamtes Wasser
3. Temperatur
4. Reaktionszeit

Die Ergebnisse sind in Tabelle I zusammengefaßt.

Tabelle 1

| Beispiel Nr. | Reaktionsbedingungen | | | | Ausbeute | | | Qualität (Gehalte)*) | |
|---|---|---|---|---|---|---|---|---|---|
| | Molverhältnis NaOH zu Melansäure/T-Säure-Isomerengemisch (MG 383) | NaOH-Konzentration bezogen auf Wasser Gew.-% | Temperatur °C | Zeit Std. | K-Säure/ H-Säure (durch Diazotierung) bezogen auf Melansäure/ T-säure-Isomerengemisch Mol-% | K-Säure (durch HFC) bezogen auf Melansäure Mol-% | H-Säure (durch HFC) bezogen auf T-Säure Mol-% | K-Säure Mg 319 % | H-Säure Mg 319 % |
| 3a | 6 : 1 | 30 | 170 | 18 | 56 | 71 | 62 | 72,4 | 30,1 |
| 3b | 7,5 : 1 | 25 | 190 | 4 | 54 | 70 | 60 | 75,0 | 32,0 |
| 3c | 5 : 1 | 35 | 180 | 8 | 53 | 68 | 61 | 74,5 | 32,5 |
| 3d | 6,5 : 1 | 30 | 210 | 1 | 54 | 70 | 60 | 73,5 | 29,9 |

*) Die Gehalte der angegebenen organischen Säuren sind auf die freie Säure berechnet. Tatsächlich liegen diese in Form der in Beispiel 1 angegebenen Salze vor. Der Gehalt an Nebenprodukten in der isolierten H-Säure und K-Säure entspricht Beispiel 1. Ergänzend zu 100% liegt praktisch nur noch Wasser und Natriumsulfat vor.

## Beispiel 4

733 g Melansäure/T-Säure-Isomerengemisch in Form der Trikaliumsalze (Gehalt 9,41 g Nitrit/100 g, 26,9 Gew.-% Melansäure MG 383; insgesamt 69 g Nitrit, 0,51 Mol Melansäure) folgender Zusammensetzung:

| | |
|---|---|
| 1-Naphthylamin-4,6,8-trisulfonsäure | 51,4% |
| 1-Naphthylamin-3,6,8-trisulfonsäure | 30,8% |
| 2-Naphthylamin-4,6,8-trisulfonsäure | 8,2% |
| 1-Naphthylamin-2,5,7-trisulfonsäure | 1,2% |
| 1-Naphthylamin-3,5,7-trisulfonsäure | 2,5% |
| 2-Naphthylamin-3,6,8-trisulfonsäure | 0,2% |

(%-Gehalte jeweils bezogen auf diazotierbare Substanz) das zusätzlich

0,5 Gew.-% 2-Naphthylamin-4,8-disulfonsäure-dinatriumsalz
0,1 Gew.-% 1-Naphthylamin-4,8-disulfonsäure-dinatriumsalz
3,0 Gew.-% Wasser

und quantitativ nicht bestimmbare Mengen an Amino- und Nitroderivaten der Dinaphthylsulfon-sulfosäuren und an Oxidationsprodukten des Naphthalins und der Naphthalintrisulfonsäuren enthält und 780 g Wasser werden in einem 2,7 l Nickel-Autoklav auf 180°C aufgeheizt. 600 g 70gew.-%ige Kalilauge (7,5 Mol KOH) von 170°C werden mit Stickstoff eingedrückt. Dabei stellt sich eine Temperatur von 190°C ein und es resultiert eine — bezogen auf das gesamte Wasser — 30gew.-%ige KOH-Lösung.

Die Reaktionsmischung wird 180 Minuten bei 190°C gehalten, mittels Kühlschlange in ca. 2 Minuten auf 150°C abgekühlt und nach weiterem Abkühlen simultan mit ca. 1400 g 50gew.-%iger $H_2SO_4$ pH-kontrolliert bei pH 1 bis 1,5 und 80 bis 90°C in eine Vorlage von 4500 g heißem Wasser einlaufen gelassen. Die Reaktionsmischung wird zur Entfernung von Schwefeldioxid 2 Stunden bei 80 bis 90°C gerührt, unter Verdampfungskühlung auf 20°C abgekühlt und 12 Stunden bei 20°C gehalten. Das Produkt wird filtriert und mit insgesamt 600 g einer ca. 10gew.-%igen wäßrigen Kaliumsulfat-Lösung gewaschen.

Die durch Diazotierung bestimmte Ausbeute beträgt 50% auf den Nitrit-Gehalt des eingesetzten Melansäure/T-Säure-Isomerengemisches bezogen. Die Zusammensetzung des K-Säure/H-Säure-Monokaliumsalz-Gemisches wurde durch Hochdruck-Flüssigkeits-Chromatographie wie folgt ermittelt:

| | |
|---|---|
| K-Säure-Monokaliumsalz | 33,6 Gew.-% |
| H-Säure-Monokaliumsalz | 30,7 Gew.-% |
| Iso-K-Säure-Monokaliumsalz | 0,1 Gew.-% |
| W-Säure-Monokaliumsalz | 0 Gew.-% |
| Melansäure-Dikaliumsalz | 0 Gew.-% |
| T-Säure-Dikaliumsalz | 0 Gew.-% |
| Dioxi-K-Säure-Dikaliumsalz | 0 Gew.-% |
| Chromotrop-Säure-Dikaliumsalz | 0,6 Gew.-% |
| Wasser | 2,0 Gew.-% |
| Kaliumsulfat | 33,2 Gew.-% |

## Beispiel 5
### (Vergleichsbeispiel)

Eine wie in Beispiel 1 durchgeführte Reaktion, jedoch unter Einsatz von reinem Melansäure-trinatriumsalz ergibt eine Ausbeute an K-Säure von 74%, jedoch einen wesentlich höheren Gehalt an Iso-K-Säure.

Gehalte im isolierten Produkt:

| | |
|---|---|
| K-Säure-Mononatriumsalz | 66,4% |
| Iso-K-Säure-Mononatriumsalz | 6,5% |
| Dioxi-K-Säure-Dinatriumsalz | 0,2% |
| Melansäure-Dinatriumsalz | 0,1% |
| Wasser | 10,2% |
| Natriumsulfat | 16,8% |

0 009 743

**Patentansprüche**

1. Verfahren zur Herstellung von 1-Amino-8-naphthol-4,6-disulfonsäure-monoalkalisalz (K-Säuremonoalkalisalz) durch alkalische Druckhydrolyse von 1-Naphthylamin-4,6,8-trisulfonsäure (Melansäure), dadurch gekennzeichnet, daß man ein Naphthylamintrisulfonsäure-Isomeren-Gemisch, wie es bei der technischen Herstellung von Melansäure anfällt und das über 40 Gew.-% 1-Naphthylamin-4,6,8-trisulfonsäure (bezogen auf die Gesamtmenge diazotierbarer Substanzen gerechnet mit Molekulargewicht 383) enthält, und/oder deren Salze mit Alkalihydroxid-Lösung bei erhöhtem Druck und erhöhter Temperatur umsetzt und anschließend durch Ansäuern und Kristallisation die 1-Amino-8-naphthol-3,6-disulfonsäure (H-Säure) und die 1-Amino-8-naphthol-4,6-disulfonsäure jeweils in Form ihrer Monoalkalisalze isoliert.

2. Verfahren nach Anspruch 1, dadurch gekennzeichnet, daß man die Reaktion in 10 bis 50gew.-%iger wäßriger Alkalihydroxid-Lösung, bezogen auf die Summe Alkalihydroxid plus gesamtes Wasser, durchführt.

3. Verfahren nach Ansprüchen 1 und 2, dadurch gekennzeichnet, daß man pro Mol diazotierbare Substanz 3,5 bis 12 Mol Alkalihydroxid einsetzt.

4. Verfahren nach Ansprüchen 1 bis 3, dadurch gekennzeichnet, daß man zur Abscheidung der Monoalkalisalze der 1-Amino-8-naphthol-3,6-disulfonsäure und der 1-Amino-8-naphthol-4,6-disulfonsäure mit Mineralsäuren einen pH-Wert im Bereich 0 bis 4 einstellt und bezogen auf das Gewicht des in der Druckhydrolyse vorliegenden Gemisches die 0,1- bis 5fache Menge Wasser einbringt.

5. Verfahren nach Ansprüchen 1 bis 4, dadurch gekennzeichnet, daß man die Isolierung des Monoalkalisalzes der 1-Amino-8-naphthol-3,6-disulfonsäure (H-Säure) bei Temperaturen im Bereich von 40 bis 100°C durchführt.

6. Verfahren nach Ansprüchen 1 bis 5, dadurch gekennzeichnet, daß man die Isolierung des Monoalkalisalzes der 1-Amino-8-naphthol-3,6-disulfonsäure (H-Säure) bei Temperaturen im Bereich von 50 bis 90°C durchführt.

7. Verfahren nach Ansprüchen 5 und 6, dadurch gekennzeichnet, daß man nach Isolierung des Monoalkalisalzes der 1-Amino-8-naphthol-3,6-disulfonsäure (H-Säure) die Isolierung des Monoalkalisalzes der 1-Amino-8-naphthol-4,6-disulfonsäure (K-Säure) bei Temperaturen im Bereich von −5 bis 40°C durchführt.

8. Verfahren nach Ansprüchen 5 bis 7, dadurch gekennzeichnet, daß man nach Isolierung des Monoalkalisalzes der 1-Amino-8-naphthol-3,6-disulfonsäure (H-Säure) die Isolierung des Monoalkalisalzes der 1-Amino-8-naphthol-4,6-disulfonsäure (K-Säure) bei Temperaturen im Bereich von 5 bis 25°C durchführt.

9. Verfahren nach Ansprüchen 1 bis 8, dadurch gekennzeichnet, daß man die Isolierung des Gemisches der Monoalkalisalze der 1-Amino-8-naphthol-3,6- und -4,6-disulfonsäure (H- und K-Säure) bei Temperaturen im Bereich von −5 bis 40°C durchführt, anschließend das Gemisch in Wasser und/oder wäßriger Alkalisulfat-Lösung, gegebenenfalls unter Zusatz geringer Mengen an Mineralsäure, suspendiert, das Monoalkalisalz der 1-Amino-8-naphthol-4,6-disulfonsäure (K-Säure) bei Temperaturen im Bereich von 40 bis 100°C in Lösung bringt, das Monoalkalisalz der 1-Amino-8-naphthol-3,6-disulfonsäure (H-Säure) abtrennt und aus dem Filtrat das Monoalkalisalz der 1-Amino-8-naphthol-4,6-disulfonsäure (K-Säure) bei Temperaturen im Bereich von −5 bis 40°C abscheidet.

**Claims**

1. A process for the production of 1-amino-8-naphthol-4,6-disulfonic acid monoalkali salt (K-acid monoalkali salt) by the alkaline pressure hydrolysis of 1-naphthylamine-4,6,8-trisulfonic acid (melan acid), characterised in that a naphthylamine trisulfonic acid isomer mixture, of the type formed in the commercial production of melan acid and containing more than 40% by weight of 1-naphthylamine-4,6,8-trisulfonic acid (based on the total quantity of diazotisable substances having a calculated molecular weight of 383), and/or salts thereof are reacted with alkali hydroxide solution at elevated temperature and pressure, after which 1-amino-8-naphthol-3,6-disulfonic acid (H-acid) and 1-amino-8-naphthol-4,6-disulfonic acid are respectively isolated in the form of their monoalkali salts by acidification and crystallisation.

2. A process as claimed in Claim 1, characterised in that the reaction is carried out in 10 to 50% by weight aqueous alkali hydroxide solution, based on the sum of alkali hydroxide plus total water.

3. A process as claimed in Claims 1 and 2, characterised in that from 3.5 to 12 moles of alkali hydroxide are used per mole of diazotisable substance.

4. A process as claimed in Claims 1 to 3, characterised in that, in order to separate off the monoalkali salts of the 1-amino-8-naphthol-3,6-disulfonic acid and the 1-amino-8-naphthol-4,6-disulfonic acid, a pH-value in the range from 0 to 4 is adjusted with mineral acids and from 0.1 to 5 times the quantity of water — based on the weight of the mixture undergoing pressure hydrolysis — is introduced.

5. A process as claimed in Claims 1 to 4, characterised in that isolation of the monoalkali salt of

10

0 009 743

1-amino-8-naphthol-3,6-disulfonic acid (H-acid) is carried out at temperatures in the range from 40 to 100°C.

6. A process as claimed in Claims 1 to 5, characterised in that isolation of the monoalkali salt of 1-amino-8-naphthol-3,6-disulfonic acid (H-acid) is carried out at temperatures in the range from 50 to 90°C.

7. A process as claimed in Claims 5 and 6, characterised in that, after isolation of the monoalkali salt of 1-amino-8-naphthol-3,6-disulfonic acid (H-acid), isolation of the monoalkali salt of 1-amino-8-naphthol-4,6-disulfonic acid (K-acid) is carried out at temperatures in the range from −5 to 40°C.

8. A process as claimed in Claims 5 to 7, characterised in that, after isolation of the monoalkali salt of 1-amino-8-naphthol-3,6-disulfonic acid (H-acid), isolation of the monoalkali salt of 1-amino-8-naphthol-4,6-disulfonic acid (K-acid) is carried out at temperatures of from 5 to 25°C.

9. A process as claimed in Claims 1 to 8, characterised in that isolation of the mixture of the monoalkali salts of 1-amino-8-naphthol-3,6- and -4,6-disulfonic acid (H-and K-acid) is carried out at temperatures in the range from −5 to 40°C, after which the mixture is suspended in water and/or aqueous alkali sulfate solution, optionally in the presence of small quantities of mineral acid, the monoalkali salt of 1-amino-8-naphthol-4,6-disulfonic acid (K-acid) is dissolved at temperatures in the range from 40 to 100°C, the monoalkali salt of 1-amino-8-naphthol-3,6-disulfonic acid (H-acid) is separated off and the monoalkali salt of 1-amino-8-naphthol-4,6-disulfonic acid (K-acid) is separated off from the filtrate at temperatures in the range from −5 to 40°C.

**Revendications**

1. Procédé de préparation de sels monoalcalins d'acide 1-amino-8-naphtol-4,6-disulfonique (sels monoalcalins d'acide K) par hydrolyse alcaline sous pression d'acide 1-naphtylamino-4,6,8-trisulfonique (acide mélanique), caractérisé en ce qu'on fait réagir un mélange d'isomères d'acides naphtylamino-trisulfoniques, tel que celui obtenu lors de la préparation d'acide mélanique à l'échelle industrielle et qui contient plus de 40% en poids d'acide 1-naphtylamino-4,6,8-trisulfonique (rapportés à la quantité totale de substances diazotables et en tenant compte d'un poids moléculaire de 383), et/ou les sels de cet acide avec une solution d'un hydroxyde alcalin sous forte pression et à température élevée puis, par acidification et cristallisation, on isole l'acide 1-amino-8-naphtol-3,6-disulfonique (acide H) et l'acide 1-amino-8-naphtol-4,6-disulfonique chacun sous forme de leurs sels monoalcalins.

2. Procédé suivant la revendication 1, caractérisé en ce qu'on effectue la réaction dans une solution aqueuse à 10−50% en poids d'un hydroxyde alcalin, calculés sur la somme de l'hydroxyde alcalin plus la quantité totale d'eau.

3. Procédé suivant les revendications 1 et 2, caractérisé en ce que, par mole de substance diazotable, on utilise 3,5 à 12 moles d'un hydroxyde alcalin.

4. Procédé suivant les revendication 1 à 3, caractérisé en ce que, pour séparer les sels monoalcalins de l'acide 1-amino-8-naphtol-3,6-disulfonique et de l'acide 1-amino-8-naphtol-4,6-disulfonique, on règle un pH se situant dans le domaine de 0 à 4 avec des acides minéraux et, en se rapportant au poids du mélange présent lors de l'hydrolyse sous pression, on introduit de l'eau en une quantité 0,1 à 5 fois plus élevée.

5. Procédé suivant les revendications 1 à 4, caractérisé en ce qu'on effectue l'isolation du sel monoalcalin de l'acide 1-amino-8-naphtol-3,6-disulfonique (acide H) à des températures se situant dans l'intervalle allant de 40 à 100°C.

6. Procédé suivant les revendications 1 à 5, caractérisé en ce qu'on effectue l'isolation du sel monoalcalin de l'acide 1-amino-8-naphtol-3,6-disulfonique (acide H) à des températures se situant dans l'intervalle allant de 50 à 90°C.

7. Procédé suivant les revendications 5 et 6, caractérisé en ce que, après isolation du sel monoalcalin de l'acide 1-amino-8-naphtol-3,6-disulfonique (acide H), on effectue l'isolation du sel monoalcalin de l'acide 1-amino-8-naphtol-4,6-disulfonique (acide K) à des températures se situant dans l'intervalle allant de −5 à 40°C.

8. Procédé suivant les revendications 5 à 7, caractérisé en ce que, après isolation du sel monoalcalin de l'acide 1-amino-8-naphtol-3,6-disulfonique (acide H), on effectue l'isolation du sel monoalcalin de l'acide 1-amino-8-naphtol-4,6-disulfonique (acide K) à des températures se situant dans l'intervalle allant de 5 à 25°C.

9. Procédé suivant les revendications 1 à 8, caractérisé en ce qu'on effectue l'isolation du mélange des sels monoalcalins de l'acide 1-amino-8-naphtol-3,6-disulfonique et de l'acide 1-amino-8-naphtol-4,6-disulfonique (acide H et acide K) à des températures se situant dans l'intervalle allant de −5 à 40°C, puis on met le mélange en suspension dans l'eau et/ou une solution aqueuse d'un sulfate alcalin en ajoutant éventuellement de faibles quantités d'un acide minéral, on dissout le sel monoalcalin de l'acide 1-amino-8-naphtol-4,6-disulfonique (acide K) à des températures se situant dans l'intervalle allant de 40 à 100°C, on sépare le sel monoalcalin de l'acide 1-amino-8-naphtol-3,6-disulfonique (acide H) et, du filtrat, on sépare le sel monoalcalin de l'acide 1-amino-8-naphtol-4,6-disulfonique (acide K) à des températures se situant dans l'intervalle allant de −5 à 40°C.

11